# EUROPEAN PATENT SPECIFICATION

(11) **EP 1 468 064 B1**
(45) Date of publication and mention of the grant of the patent: **23.07.2008**
(21) Application number: 03707484.6
(22) Date of filing: 23.01.2003
(51) Int. Cl.: C11B 9/00, C07D 311/00, A61Q 13/00

(54) **FRAGRANCE COMPOUND**
RIECHSTOFFVERBINDUNG
COMPOSE DE FRAGRANCE

(30) Priority: 23.01.2002 US 351313 P
(43) Date of publication of application: 20.10.2004
(73) Proprietor: E.I. DU PONT DE NEMOURS AND COMPANY, Wilmington DE 19898 (US)
(72) Inventor: HALLAHAN, David, L., Wilmington, DE 19808 (US)
(74) Representative: Towler, Philip Dean
(86) International application number: PCT/US2003/001936
(87) International publication number: WO 2003/062357

(56) References cited:
- WO-A-98/27261
- J. WOLINSKY ET AL : "Syntheses of the dihydronepetalones" JOURNAL OF ORGANIC CHEMISTRY. , vol. 37, no. 21, 1972, pages 3376-3378, XP002242594 AMERICAN CHEMICAL SOCIETY. EASTON.; US cited in the application
- T. SAKAN ET AL : "On the structure of actinidine and matatabilactone, the effective components of actinidia polygama." BULLETIN OF THE CHEMICAL SOCIETY OF JAPAN , vol. 32, 1959, pages 315-316, XP002242595 JAPAN PUBLICATIONS TRADING CO. TOKYO.; JP

## Description

### FIELD OF THE INVENTION

The present invention relates to the field of perfumery and aroma, and the use of dihydronepetalactone stereoisomers generally as fragrance or aroma materials. These chemicals may be prepared by extraction of herbaceous material, entirely synthetically, or semi-synthetically using as starting material a processed extract of herbaceous plants containing as starting material nepetalactones.

### BACKGROUND OF THE INVENTION

A wide variety of chemicals, both natural and synthetic, are known to possess organoleptic effects in humans. Of these, a small proportion possess pleasing fragrance notes and are used commercially as fragrant or aroma materials. There is an on-going need to develop new fragrance and aroma materials that can be synthesized from relatively inexpensive raw materials. There are several reasons for this, notably toxicological constraints, environmental considerations, biodegradability, performance, and cost effectiveness. While all of these factors must be carefully weighed in consideration of whether to introduce a new fragrance material, perhaps the most important factors are performance and cost. Performance properties include odor activity, notes, and aesthetics; substantivity; and solubility. The cost effectiveness involves manufacture costs and the amount of the compound required to impart fragrance to a consumable product. Of course, the lower the amount of fragrance material required, the higher its cost effectiveness. Many materials have met some of the above-mentioned criteria, yet have not been successful because of disappointing ratios of cost versus performance. Lastly, consideration must be given to the rigid regulatory positions in many countries governing use of ingredients in consumable products.

Many herbaceous plant species produce aromatic oils (essential oils) which are used as natural sources of fragrant chemicals (Hay, R.K.M., Svoboda, K.P. Botany in 'Volatile Oil Crops: their biology, chemistry and production'. Hay, R.K.M., Waterman, P.G. (eds.); Longman Group UK Limited (1993)). Examples include *Melissa officinalis* (Melissa), *Perilla frufescens* (Perilla), *Posostemon cablin* (Patchouli) and various *Lavandula* spp. (Lavender). All of these examples are members of the Labiatae (Lamiaceae) family, Plants of the genus *Nepeta* (catmints) are also members of this family, and produce an essential oil which is a minor item of commerce. This oil is very rich in a class of monoterpenoid compounds known as iridoids [Inouye, H. Iridoids. Methods in Plant Biochemistry 7:99-143 (1991)]. more specifically the methylcyclopentanoid nepetalactones (Clark, L.J. et al. The Plant Journal, 11:1387-1393 (1997)) and derivatives.

Four stereoisomers of nepetalactone are known to exist in nature. These chemicals exert a well-known excitatory effect on cats [Sakurai et al. Agric. Biol. Chem. 52(9): 2369-71 (1988)] and thus the oil--or more commonly, the dried herbage of this plant termed catnip-is used in cat toys. The leaves and oil of *Nepeta* spp. do not possess a particularly attractive aroma. The uses of the herbage and oil has therefore been confined to the small market offered by domestic cat toys and accessories. A small proportion of the oil of various *Nepeta* spp. consists of dihydronepetalactones, which are possibly derived biosynthetically from the more abundant nepetalactones [Regnier, F.E., et al. Phytochemistry 6:1281-1289 (1967); DePooter, H.L., et al. Flavour and Fragrance Journal 3:155-159 (1988); Handjieva, N.V. and S.S. Popov. J. Essential Oil Res. 8:639-643 (1896)]. In contrast to the many studies carried out with nepetalactones, the human organoleptic properties of dihydronepetalactones have not been investigated or disclosed in the literature. The compounds thus form a class of fragrant molecules the use of which has never been proposed in perfumery.

WO 98/27261 discloses animal care systems and animal litter comprising perfumes, which may comprise nepetalactones.

There Is an on-going need for new fragrance materials that can be readily synthesized from relatively inexpensive raw natural materials and can meet the criteria set forth above, which include possession of unique fragrance notes and cost-effective production. It has been found that a suite of dihydronepetalactone compounds, defined according to the structure shown in Formula 1 below, possess many properties that make it desirable for use as a fragrance compound or perfume by itself or in combination with other materials. The dihydronepetalactones may be delivered in a variety of forms for application to the skin or in an article of manufacture.

These compounds may be isolated from plants of the genus *Nepeta*, and possess a unique and pleasing fragrance.

### SUMMARY OF THE INVENTION

It is within the scope of the invention to provide a non-therapeutic method of treating skin comprising applying thereto a dihydronepetalactone, or a mixture of dihydronepetalactone stereoisomers, represented by the general formula: 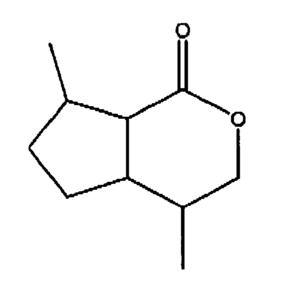 The dihydronepetalactones have a unique and pleasing fragrance. In a preferred embodiment the dihydronepetalactone stereoisomers are (7*S*)-dihydronepetalactone stereoisomers derived from (7*S*)-nepetalactones.

### BRIEF DESCRIPTION OF DRAWINGS

Figure 1 shows the chemical structure of the naturally-occurring iridoid (methylcyclopentanoid) nepetalactones.
Figure 2 shows the total ion chromatograms from combined gas chromatography/mass spectrometry (GC-MS) analysis of a distilled nepetalactone-enriched fraction from commercially-available catmint oil together with that of the material produced from this fraction by hydrogenation.
Figure 3 shows the mass spectra of the major constituents of the nepetalactone-enriched fraction and the hydrogenated material identified by GC-MS analysis.
Figure 4 shows the ¹³C NMR analysis performed on a distilled nepetalactone-enriched fraction of commercially-available catmint oil.
Figure 5 shows the ¹³C NMR spectrum obtained from analysis of the dihydronepetalactones produced by hydrogenation of a distilled nepetalactone-enriched fraction of commercially-available catmint oil.

### DETAILED DESCRIPTION OF THE INVENTION

In this disclosure, as a number of terms and abbreviations are used, the following definitions are provided to further understanding of the invention.

The term "nepetalactone" refers to the compound having the general structure:

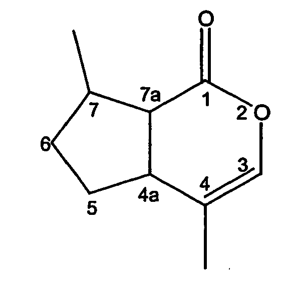

Four chiral centers are present within the methylcyclopentanoid backbone of nepetalactone at carbons 4, 4a, 7 and 7a as shown above; (7S)-nepetalactones are produced by many plants and insects.

The term "dihydronepetalactones" or "dihydronepetalactone mixtures" refers to any mixture of dihydronepetalactone stereoisomers. The molar or mass composition of each of these isomers relative to the whole dihydronepetalactone composition can be variable. Dihydronepetalactones are defined by Formula 1: 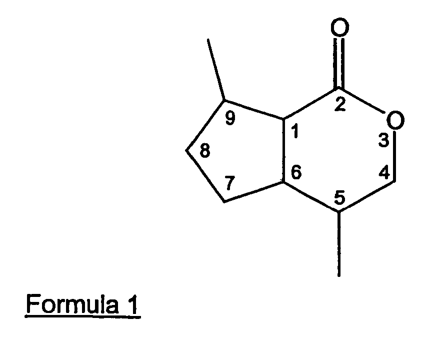 wherein 1, 5, 6 and 9 indicate the four chiral centers of the molecule and the structure encompasses all possible stereoisomers of dihydronepetalactone. The structures of dihydronepetalactone stereoisomers that may be derived from (7*S*)-nepetalactones are shown below.

| | |
|---|---|
| 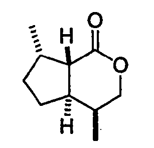 | (1S,5S,9S,6R)-5,9-dimethyl-3-oxabicyclo[4.3.0]nonan-2-one |
| 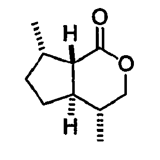 | (1S,9S,5R,6R)-5,9-dimethyl-3-oxabicyclo[4.3.0]nonan-2-one |
| 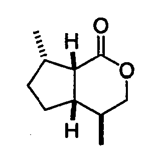 | (1S,5S,9S,6S)-5,9-dimethyl-3-onabicyclo[4.3.0]nonan-2-one |
| 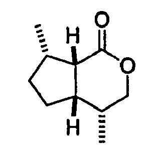 | (1S,9S,6S,SR)-5,9-dimethyl-3-oxabicyclo[4.3.0]nonan-2-one |
| 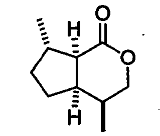 | (9S,5S,1R,6R)-5,9-dimethyl-3-oxabicyclo[4.3.0]nonan-2-one |
| 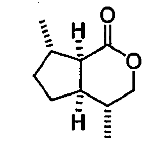 | (9S,1R,5R,6R)-5,9-dimethyl-3-oxabicyclo[4.3.0]nonan-2-one |
| 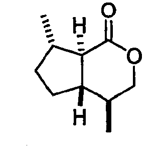 | (9S,6S,1R,5S)-5,9-dimethyl-3-oxabicyclo[4.3.0]nonan-2-one |
| 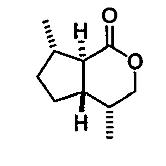 | (9S,6S,1R,5R)-5,9-dimethyl-3-oxabicyclo[4.3.0]nonan-2-one |

As used herein, the terms "alter" and "modify" in their various forms refer to a means of supplying or imparting a fragrance, aroma character or note to otherwise bland substances, or augmenting existing aroma characteristics where natural aroma is deficient in some regard, or supplementing an existing aroma impression to modify its quality, character, or aroma.

The term "enhance" is intended to mean the intensification (without effecting a change in kind or quality of aroma) of one or more aroma nuances and their organoleptic impression of a fragrance, perfume composition, or one or more perfumed articles.

The term "fragrance note(s)" or "note(s)" refers to the three stages that most fragrances go through. The top note is the first impression of the fragrance. The middle note is the main character of a fragrance. These are stronger, mid-range notes that emerge after the top and linger longest, as the 'heart' of the fragrance. Finally, the base note is the final scent of the fragrance. These rich, heavy notes emerge slowly and definitely, echoing resonantly after the others die down. Bottom notes, by definition, linger behind and act as a fixative to stop the lighter oils from dispersing too quickly.

The terms "perfume composition" or "fragrance composition" or "aroma composition" are used herein to mean a mixture of organic compounds including, for example, alcohols, aldehydes, ketones, nitriles, esters, lactones, natural essential oils, synthetic essential oils, and mercaptans, which are admixed so that the combined odors of the individual components produce a pleasant or desired fragrance. Such compositions usually contain: (1) the main note or the "bouquet" or foundation stone of the composition; (2) modifiers which round off and accompany the main note; (3) fixatives which include odorous substances which lend a particular note to the perfume throughout all stages of evaporation and substances which retard evaporation; and (4) top notes which are usually low-boiling, fresh-smelling materials.

A perfume is characterized by its uniquely pleasing fragrance or aroma. In perfume, fragrance or aroma compositions, the individual component will contribute its particular olfactory characteristics, but the overall effect of the composition will be the sum of each of the effects of each of the ingredients. Thus, the dihydronepetalactones of this invention or mixtures thereof can be used to alter the aroma characteristics of such compositions, for example, by highlighting or moderating the olfactory reaction contributed by another ingredient in the composition.

A "perfume composition" may contain a perfume compound, a fragrance compound or an aroma compound in addition to other materials.

### Isolation and Synthesis of Dihydronepetalactones

Dihydronepetalactones are known in the literature, as minor constituents of the essential oils of several labiate plants of the genus *Nepeta* (Regnier, F.E., et al. Phytochemistry 6:1281-1289 (1967); DePooter, H.L., et al. Flavour and Fragrance Journal 3:155-159 (1988); Handjieva, N.V. and S.S. Popov J. Essential Oil Res. 8:639-643 (1996)). They have also been identified as constituents of the defensive secretions of certain insects, including rove beetles (Jefson, M., et al. J. Chem. Ecol. 9:159-180 (1983)) and ants, specifically *Iridomyrmex* species (Cavill, G.W.K. and D.V. Clark. J. Insect Physiol. 13:131-135 (1967)).

Although the roles of dihydronepetalactones produced in plants remain unknown, investigations of their roles in insects indicates an involvement with repulsing predators (Jefson, M., et al. J. Chem. Ecol. 9:159-180 (1983); Cavill, G.W.K., and D.V. Clark. J. Insect Physiol. 13:131-135 (1967)). Nothing, however, explicitly or implicitly discloses the unique utility of dihydronepetalactone mixtures for augmenting, enhancing or imparting fragrance properties.

The chemical synthesis of dihydronepetalactones and their related iridoid monoterpenoid compounds has been described and found to be conducted in a variety of ways. The following are useful references relating to synthesis:
1) Abelman, M.M. et al. J. Am. Chem. Soc. 104(14):4030-2 (1982)
2) Fleming, I. and N.K. Terrett. Tetrahedron Lett. 25(44): 5103-5104 (1984); J. Chem. Soc., Perkin Trans. 1:2645-2650 (1998).
3) Lee, E. and C.H. Yoon. J. Chem. Soc., Chem. Commun. 4: 479-81 (1994).
4) Nagata, H. and K. Ogasawara. Tetrahedron Lett. 40(36): 6617-6620 (1999).
5) Nangia, A. et al. Tetrahedron Lett. 35(22): 3755-8 (1994).
6) Tanimori, S. and M. Nakayama. Agric. Biol. Chem. 55(4): 1181-1184 (1991).
7) Uyehara, T. et al. J. Chem. Soc., Chem. Commun. 2:113-14 (1989); Tennen Yuki Kagobutsu Toronkai Koen Yoshishu 32; 441-6 (1890); J. Org. Chem. 57(11): 3139-3145 (1992).
8) Wolinsky, J. and E.J. Eustace. J. Org. Chem. 37(21):3376-8 (1972).
9) Wolinsky, J. and D.L. Nelson. Tetrahedron 25(17): 3767-74 (1969).

One preferred and convenient method for the synthesis of dihydronepetalactone mixtures as used in the method of the present invention is by hydrogenation of nepetalactone. Catalysts such as platinum oxide and palladium supported on strontium oxide give dihydronepalactone in 24-90% yields (Regnier, F.E., et al. Phytochemistry 6:1281-1289 (1967)). Nepetalactone is a known material that can be conveniently obtained in relatively pure form from the essential oils isolated by various means from plants of the genus *Nepeta* (catmints). Isolation of such oils is well known in the art, and examples of methodology for oil extraction include (but are not limited to) steam distillation, organic solvent extraction, microwave-assisted organic solvent extraction, supercritical fluid extraction, mechanical extraction and enfleurage (initial cold extraction,into fats followed by organic solvent extraction).

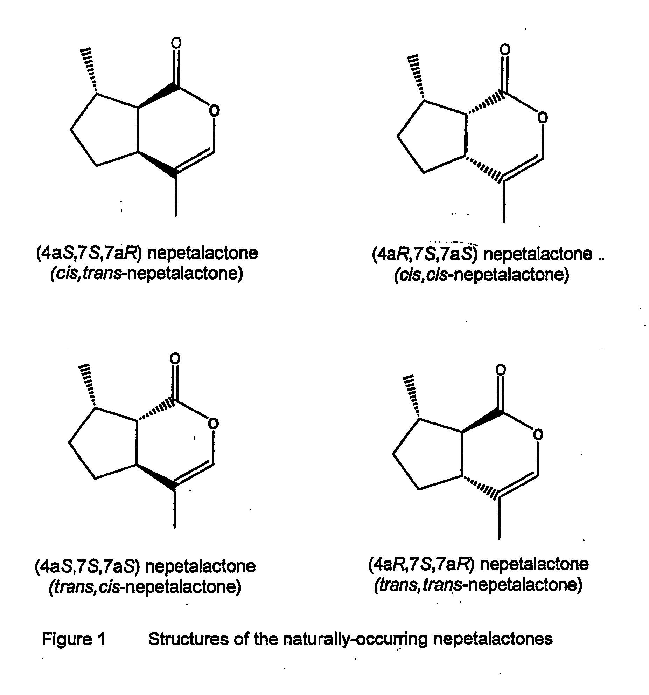

The essential oils isolated from different *Nepeta* species are well known to possess different proportions of each naturally-occurring stereoisomer of nepetalactone (Regnier. F.E., et al. Phytochemistry 6:1281-1289 (1967); DePooter, H.L., et al. Flavour and Fragrance Journal 3:155-159 (1988); Handjieva, N.V. and S.S. Popov. J. Essential Oil Res. 8:639-643 (1996)). Thus, from oil derived from any *Nepeta* species containing a mixture of nepetalactones, a mixture of dihydronepetalactone stereoisomers will be generated upon hydrogenation. Four chiral centers are present within the methylcyclopentanoid backbone of the nepetalactone at carbons 4, 4a, 7 and 7a as shown below: 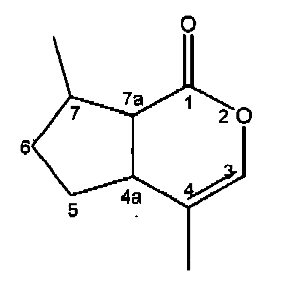 Thus it is clear that a total of eight pairs of dihydronepetalactone enantiomers are possible after hydrogenation. Of these, the naturally occurring stereoisomers described thus far are (7*S*)-dihydronepetalactones. Preferred fragrance materials used in the method of the present invention include a mixture of any or all of the possible stereoisomers of dihydronepetalactone. More preferred fragrance materials include a mixture of (7*S*)-dihydronepetalactones. Most preferred are (7*S*)-dihydronepetalactone stereoisomers derived from (7*S*)-nepetalactones. This includes the compounds commonly known as cis,trans-nepetalactone, cis,cis-nepetalactone, trans,cis- nepetalactone, and trans,trans-nepetalactone, as illustrated in Figure 1.

Upon completion of the hydrogenation reaction, the resulting mixture of isomer products may be separated by a conventional method, such as for example, by preparative liquid chromatography to yield each highly purified pair of dihydronepetalactone enantiomers. Chiral chromatography may be employed to separate enantiomers.

In addition to variation in nepetalactone stereoisomer content between different *Nepeta* species, intra-species variation is also known to exist. Plants of a given species may produce oils with different compositions depending on the conditions of their growth or growth stage at harvest. Additionally, within a single species, variation in oil composition independent of growth conditions or growth stage at harvest has been demonstrated (Clark, L.J., et al. The Plant Journal, 11:1387-1393 (1997)). Plants of a single species exhibiting different oil compositions are termed chemotypes, and it has been shown that in *Nepeta* spp., chemotypes exhibiting marked differences in the proportion of different nepetalactone stereoisomers exist (Clark, L.J.,et al., *supra).* Thus, the preferred process for producing specific dihydronepetalactone enantiomers would be hydrogenation of an oil from a *Nepeta* chemotype known to contain specific nepatelactone stereoisomers.

Therefore, the preferred process for producing the dihydronepetalactones represented by Formula I in the present invention is by hydrogenation of nepetalactones from plants with oils of defined nepetalactone stereoisomer content, an industrially advantageous approach in terms of production cost and its biological basis. Other processes are as disclosed in U.S. Provisional Application No. 60/369,470, filed April 3, 2002.

### Fragrance of Dihydronepetalactones

Dihydronepetalactones possess a unique, pleasant fragrance. A mixture of dihydronepetalactone stereoisomers, prepared as described above, was subjected to sensory analysis by commercial perfumers, limited exclusively to testing the scent. The material was found to have a minty (peppermint), fresh-like odor with a hint of pulegone (1-isopropylidene-4-methyl-2-cyclohexanone).

The unique fragrance notes of the subject compounds thus make them useful in imparting, altering, augmenting or enhancing the overall olfactory component of a perfume composition, for example, by utilizing or moderating the olfactory reaction contributed by one or more other ingredients in the composition. Specifically, the composition may be utilized to either: (1) impart a characteristic perfume or aroma to a perfume or article; or (2) mask or modify the odor of one or more of the components thereof.

As will be appreciated, fragrant materials are typically utilized in combinations that may include both natural and synthetic ingredients to achieve the desired overall perfume effect. Fragrance developers consider the scent of the compound, as well as its efficacy, degree of stability within the final formulation, activity during a product's shelf life, and lack of adverse reaction with the product or its intended function as a perfumed article. Typically, fragrances are used to mask the odor contributed by other ingredients in the formulation of the final scented product, and/ or to enhance consumer appeal of a product.

### Perfume Compositions and Articles

It is expected that the dihydronepetalactone mixtures may be modified with a variety of formulations, carriers, ingredients, or other moieties that will comprise a perfume composition. A non-limiting list of such materials will include for example, alcohols, aldehydes, ketones, nitriles, esters, lactones, natural essential oils, synthetic essential oils and mercaptans. These materials may be admixed with the dihydronepetalactones used in the method of the present invention so that the combined odors of the individual components produce a pleasant and desired fragrance. It is to be understood that such additional adjuvants are to be organoleptically compatible with the dihydronepetalactones used in the method of this invention and further that such adjuvants are to be non-reactive under use conditions (such as at room temperature, e.g. 25C) and storage conditions with the dihydronepetalactones used in the method of this invention. The nature and the variety of these coingredients do not require a more detailed description here, which, moreover, would not be exhaustive, and the artisan will be able to choose the latter based on general knowledge and as a function of the nature of the product to be perfumed and of the desired olfactive effect. A large number of these ingredients are listed in reference textbooks such as the book of S. Arctander, Perfume and Flavor Chemicals, 1969, Montclair, NJ, or its more recent versions, or in other works of similar nature.

In a similar manner as discussed above, the perfume composition can contain a vehicle or carrier for the dihydronepetalactone mixtures alone or with other ingredients. The vehicle can be a liquid such as an alcohol such as ethanol, a glycol such as propylene glycol or 1,6 hexylene glycol, or the like. The carrier can be an absorbent solid such as a gum (e.g., guar gum, xanthan gum, or gum arabic) or components for encapsulating the composition such as gelatin (as by coacervation) or a urea formaldehyde prepolymer (to form a urea formaldehyde polymer wall around a liquid perfume center) which can be used to form a capsule wall surrounding the perfume oil.

Dihydronepetalactones possess unique fragrant notes and, therefore, are particularly useful individually and in combination with other fragrant chemicals in a variety of perfume compositions. The overall effect of the perfume composition will be the sum of the effects of each of the ingredients. Thus, the dihydronepetalactones of the invention or mixtures thereof with other perfumery materials can be used to alter the aroma characteristics of a perfume composition, for example, by highlighting or moderating the olfactory reaction contributed by another ingredient in the composition, such as a fragrance compound that is not a dihydronepetalactone. The compounds used in the method of the present invention can be used in practically every field of modem perfumery.

The dihydronepetalactones disclosed herein can be utilized to alter, modify, augment or enhance sensory properties, particularly human organoleptic properties such as fragrance, in a wide variety of consumable materials. Dihydronepetalactones can, for example, be used in functional perfumery and in the manufacture of articles such as perfumed articles. Perfumed articles will generally, but not necessarily, comprise a perfume composition of the invention, but will contain an effective amount of dihydronepetalactones, such as an amount effective to constitute or impart a pleasing fragrance. Typical articles that can be improved by the use of dihydronepetalactones and mixtures thereof include, but are not limited to, the following categories and examples of products.

### Pet Products: pet treatment products such as pet cleaning shampoos and powders.

The variety of products in which the pleasing fragrance and aroma of dihydronepetalactones may be beneficially used illustrates that yet another embodiment of this invention is a non-therapeutic method for treating, conditioning, enhancing, enriching, refreshing or toning skin. Any number of products that are or contain dihydronepetalactones such as a lotion, bath gel, body spray or perfume function as a topical treatment, conditioner, application or enhancement for skin or as a skin-care product because they may be applied by a person to his or her skin to treat, condition, refresh or enhance the skin. All of these products are desirable or are improved by the presence of the pleasing fragrance and aroma of dihydronepetalactones, whether they are present alone as a perfume or together with other components in a perfumed formulation or composition for skin treatment or care. As a consequence, dihydronepetalactones, with their pleasing fragrance and aroma, have virtually unlimited utility as a topical treatment, conditioner, application, enhancement, preparation, aid, toner, tonic, stimulant or vitalizer for skin. Examples of various types of a topical treatment for skin that is or incorporates dihydronepetalactones are personal care products such as the following: hair-care products (such as shampoos, hair tonics, pomade, lacquers, brilliantines, hair sprays, and hair rinses); skin-care products such as deodorants; a beautifying agent such as cosmetics (such as cosmetic creams and powders) or make-up; soaps; lotions; talcs and other powders; bath salts; a cleanser for bathing such as bath and shower gels and foam products; shaving foams; a body wash; a body splash; a body rub; a body spray; a lotion; a cream; an ointment; cologne; and perfume.

Dihydronepetalactones used in the method of the present invention may be utilized individually or combined in any proportion. As is conventional in the art, the desired amount of a perfume composition to be added to a given preparation or perfumed article or product is determined by the nature of the product and other factors. These factors include both considerations of cost and the nature of the other ingredients in the perfumed composition or perfumed article, their amounts, and the effect desired on the finished perfumed article and the particular fragrance sought.

The amount of each dihydronepetalactone of Formula I or mixtures thereof in a perfume, perfume composition or perfumed article used in the method of the present invention will generally not exceed about 50% by weight based on the weight of the final product, however, greater amounts may be utilized in certain applications and this amount is not limiting. Thus, a suitable amount of dihydronepetalactone will be at least 0.01% by weight of the total weight of the perfume composition, where a range of from about 0.1 % to about 95% by weight of the total weight of the perfume composition is preferred, and from about 0.01 %, or about 1%, to about 50% by weight of the total weight of the perfume composition is most preferred.

### EXAMPLES

The present invention is further defined in the following Examples. It should be understood that these Examples, while indicating preferred embodiments of the invention, are given by way of illustration only.

The meaning of abbreviations is as follows: "h" means hour(s), "min" means minute(s), "sec" means second(s), "d" means day(s), "mL" means milliliters, "L" means liters, "m/z" means mass (m) to charge (z) ratio, "ppm" means parts per million, "mol %" means percentage expressed on a molar basis, "Hz" means Hertz (1/sec), and "psig" means pounds per square inch gauge.

### EXAMPLE 1 (REFERENCE)

### Preparation of nepetalactones by steam distillation of oil of Nepeta cataria

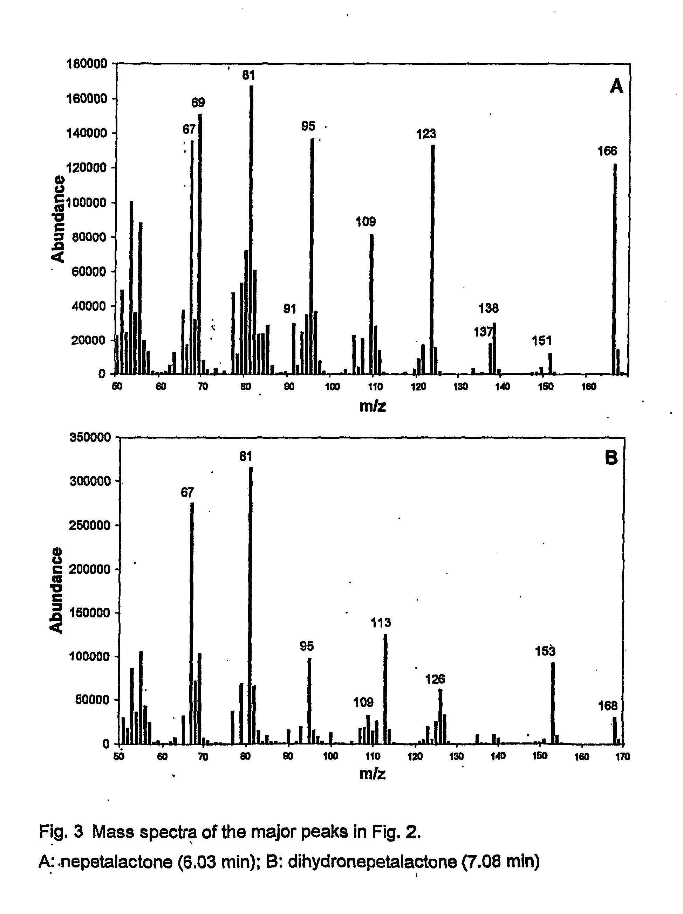

A sample of commercially-available catnip oil, prepared by steam distillation of herbaceous material from the catmint *Nepeta cataria,* was obtained (Berjé, Bloomfield, New Jersey, USA). Combined gas chromatography - mass spectrometry (GC-MS) of this oil indicated that the principal constituents were nepetalactone stereoisomers (data not shown). The nepetalactone fraction was further purified by steam distillation of this oil. GC-MS analysis of this purified fraction also indicated that it consisted predominantly of these nepetalactones (m/z 166), accompanied by trace amounts of the sesquiterpenoids caryophyllene and humulene (Fig, 2A, Fig. 3A).

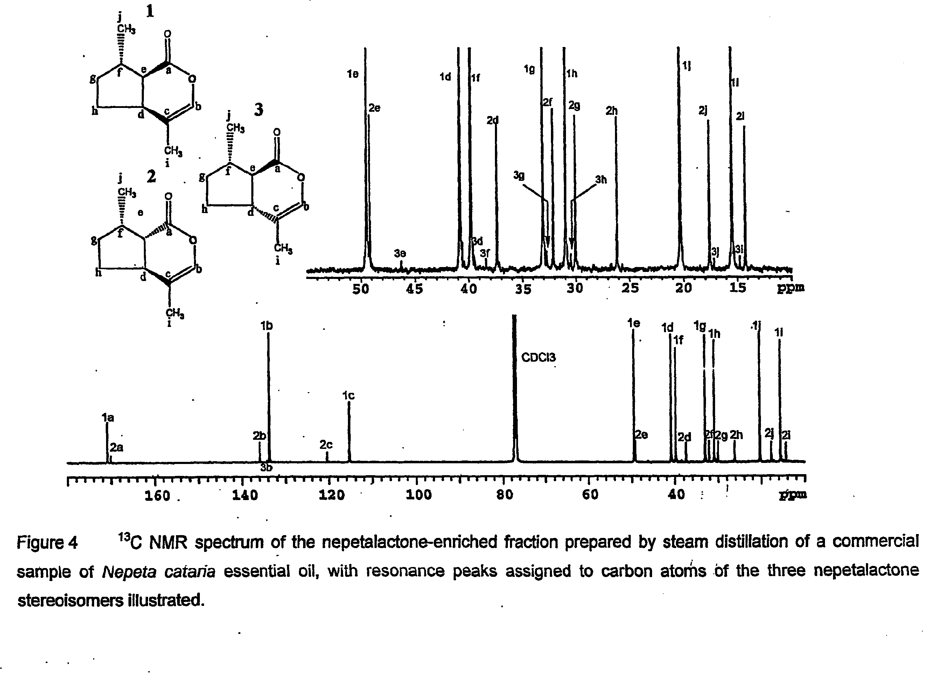

¹H and ¹³C NMR analysis of the oil and the purified material was carried out (Figure 4). Three stereoisomers, with one in major proportion, were detected in the samples. The ¹³C chemical shifts for the four possible stereoisomers reported in the literature were compared to the spectra taken for the sample. The amounts of the three components detected were quantitated, based on the carbonyl region at around 170 ppm. The chemical shifts, for both the original oil and the enriched material, are provided in Table 1. Each carbon atom of nepetalatone is identified, as shown in Figure 4.

**Table 1**

| ¹³C Chemical Shifts and Mol% Values of Nepetalactone Stereoisomers Present in the Commercial Sample of Essential Oil of Catmint (*Nepeta* *cataria*) and the Fraction Purified by Steam Distillation | | | | | | |
|---|---|---|---|---|---|---|
| ATOM | **ESSENTIAL OIL** | | | **PURIFIED FRACTION** | | |
| | *cis, trans-* | *trans, cis-* | *cis, cis-* | *cis, trans-* | *trans, cis-* | *cis, cis-* |
| | δ (ppm) | δ (ppm) | δ (ppm) | δ (ppm) | δ (ppm) | δ (ppm) |
| a | 170.9 | 170.1 | | 170.8 | 170.1 | |
| b | 133.7 | 135.9 | 134.2 | 133.7 | 135.9 | 134.2 |
| c | 115.3 | 120.4 | | 115.3 | 120.4 | |
| d | 40.8 | 37.3 | 39.6 | 40.8 | 37.4 | 39.5 |
| e | 49.4 | 49.1 | 46.4 | 49.5 | 49.1 | 46.3 |
| f | 39.7 | 32.1 | 38.4 | 39.8 | 32.1 | 38.4 |
| g | 33.0 | 30.0 | 32.7 | 33.1 | 30.0 | 32.7 |
| h | 30.9 | 26.1 | 30.4 | 31.0 | 26.1 | .30.5 |
| j | 20.2 | 17.5 | 17.1 | 20.3 | 17.6 | 17.2 |
| i | 15.4 | 14.2 | 14.7 | 15.5 | 14.2 | 14.8 |
| **Mol%** | **80.20%** | **17.70%** | **2.10%** | **84.50%** | **14.30%** | **1.20%** |

This analysis indicated that in the oil, nepetalactones were present in the following proportions: 80.2 mol% *cis,trans*-nepetalactone, 17.7 mol% *trans,cis*-nepetalactone and 2.1 mol% cis,cis-nepetalactone. The data indicated the proportions of nepetalactones in the purified material were 84.5 mol% *cis,trans*-nepetalactone, 14.3 mol% *trans,cis*-nepetalactone and 1.2 mol% *cis,cis*-nepetalactone.

### EXAMPLE 2 (REFERENCE)

### Preparation of dihydronepetalactones

107 g of the steam distilled nepetalactone fraction of the catmint oil prepared as described in Example 1 was dissolved in ethanol (200 ml) and placed in a Fisher-Porter bottle with 12.7 g 2% Pd/SrO₃. The tube was evacuated and backfilled with H₂ two times, then charged with H₂ at 30 psig (0.2 MPag). After 48 h stirring at room temperature, the tube was vented and the contents filtered over Celite to remove catalyst. The solvent was removed under vacuum, yielding a clear oil.

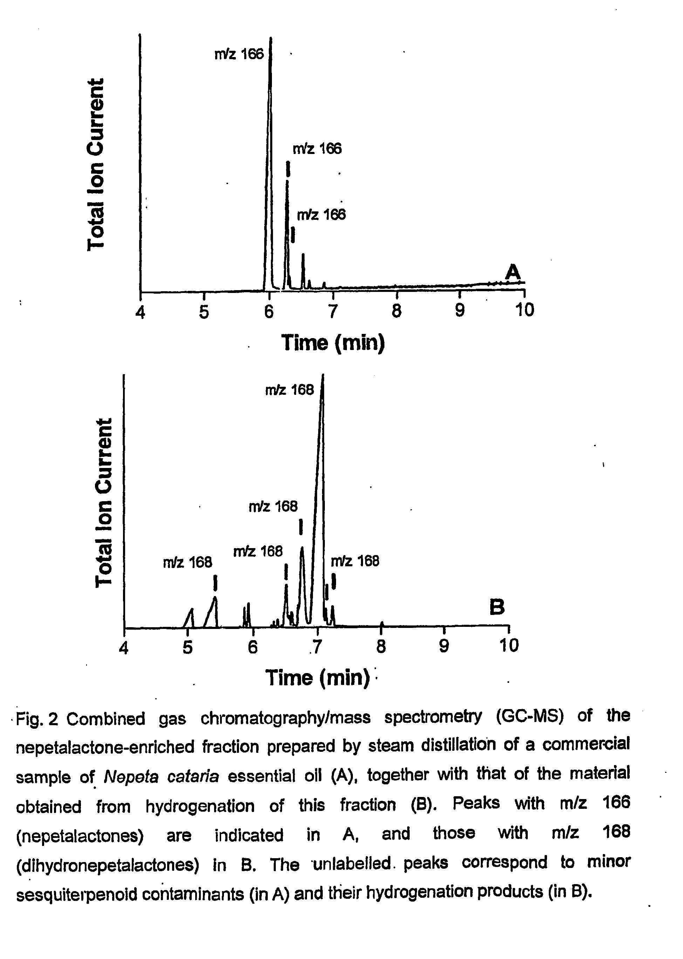

GC-MS analysis (column HP5-MS, 25 m x 0.2 mm; Oven 120°C, 2 min, 15°C/min, 210°C, 5 min.; He @ 1 ml/min) of this material indicated that the principal component (65.43% area; Rt 7.08 min) represented a dihydronepetalactone isomer, with m/z 168 (Fig. 2B). Five additional peaks, representing the remaining dihydronepetalactone enantiomers which might be derived from the three nepetalactones present in the starting material, were also represented in the chromatogram. These occurred at Rt 5.41 min, 6.8% area, m/z 168; Rt 5.93 min, area 1.2%, m/z 168; Rt 6.52 min, 4.88% area, mass 168; Rt 6.76 min, 13.8% area, m/z 168 and Rt 7.13 min, 1.25% area, m/z 168. No residual nepetalactones were detected in this analysis.

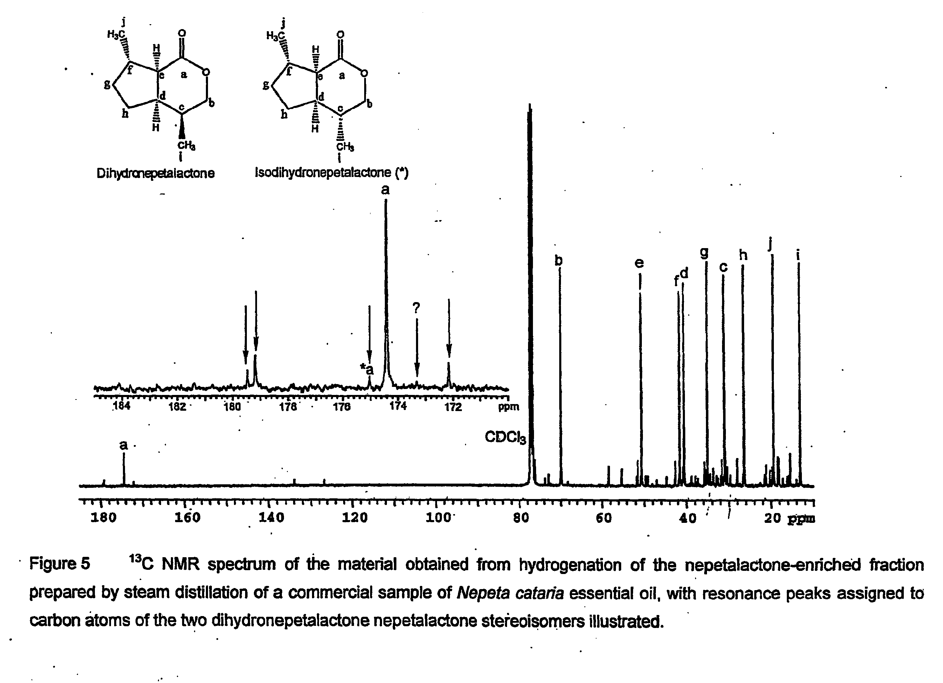

¹H, ¹³C and a series of 2D NMR methods were performed on this material. By looking at the carbonyl region of the ¹³C NMR spectrum, at least five spin systems were detected in this sample, one of them in larger amounts than the rest (ca. 75%). Examination of the ¹³C NMR spectrum (Figure 5) and comparison of chemical shifts again indicated that no residual nepetalactone was present.

Identification of the major component was done by measurement of coupling constants in the ¹H NMR spectrum and analysis of the NOESY spectrum. The coupling constants are listed in the following table (Table 2).

**Table 2**

| Coupling Constants in the ¹H NMR and NOESY Spectrum Analysis of Dihydronepetalactones produced bv Hydrogenation of Nepetalactone | | | | | | | |
|---|---|---|---|---|---|---|---|
| ATOM | ¹³C δ (PPM) | ¹H δ (PPM) | MULTIPLICITY | J (HZ) | J (HZ) | J (HZ) | J (HZ) |
| a | 174.4 | | | | | | |
| b | 70.0 | 4.1(endo) | dd | 11.1 | | 3.5 | 1.5 |
| | | 4.0(exo) | ddd | 11.1 | 10.6 | | |
| c | 31.0 | 2.2 | ddd | | 11 | 3.8 | 7.7 |
| d | 40.5 | 2.5 | dddd | 8.1 | 10.4 | | 2.2 |
| e | 50.6 | 2.4 | dd | 9.2 | 10.7 | | |
| f | 41.6 | 2 | m | | | | |
| g | 35.1 | 1.9(endo) | dddd | 11.9 | 6.1 | 6.1 | 2.5 |
| | | 1.19(exo) | dddd | 12.3 | 6.1 | 12.1 | 12 |
| h | 26.4 | 1.7(exo) | dddd | 12.9 | 7.1 | 5.9 | 1.5 |
| | | 1.4(endo) | dddd | 12.4 | 10.6 | 6.1 | 12.4 |
| i | 13.1 | 0.9 | d | 7.2 | | | |
| j | 19.4 | 1.2 | d | 6.5 | | | |

Nuclear Overhauser effects (NOE) were observed between the following protons: (1) i to d, i to c, i to h (exo), i to h (endo); (2) j to f, j to e, j to d; and (3) h (endo) to h(exo). Based on the analysis of coupling constants and the intensities of the different NOE cross peaks observed (especially the intensity of the NOE from i to d with respect to the intensity of the NOE from j to e), the stereochemistry of the principal component of the material was determined as corresponding to the dihydronepetalactone of Formula 2:

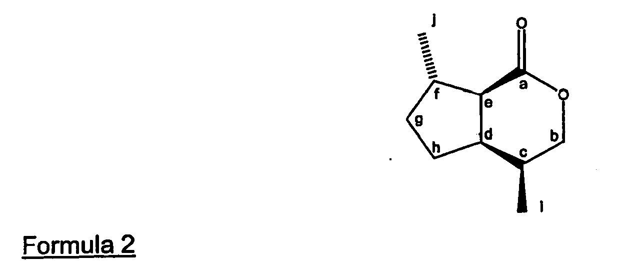

Energy minimization on a molecular model was performed and the main ¹H-¹H atomic distances are listed in Table 3.

**Table 3**

| ¹H-¹H Atomic Distances Determined via Energy Minimization | |
|---|---|
| ATOMS | DISTANCE (A) |
| i(Me)-d | 4.03 |
| j(Me)-e | 3.02 |
| i(Me)-c | 2.83 |
| j(Me)-f | 2.70 |
| j(Me)-g | 3.15 |
| j(Me)-g(exo) | 3.38 |
| i(Me)-h | 2.89 |
| i(Me)-h(exo) | 3.88 |
| i(Me)-b(endo) | 3.21 |
| i(Me)-b(exo) | 4.00 |
| i(Me)-b(exo) | 4.00 |
| i(Me)-e | 4.73 |
| j(Me)-d | 4.56 |

The intensities of the NOE cross peaks are consistent with the distances observed in the energy minimized model. The distances between the methyl groups and the adjacent protons (d and e) are different, indicating a difference in the orientation of the two methyls. The distance between the methyl group (i) and proton d is the longest, an observation consistent with the *trans* position.

The stereoisomer isodihydronepetalactone (Formula 3) was identified by ¹³C chemical shift comparison and is present at 3.6%. The other three components detected are very similar molecules but due to overlapping ¹³C chemical shifts it was not possible to conclusively identify their stereochemistry.

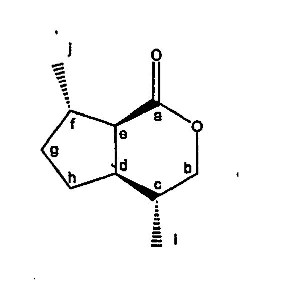

### Formula 3

Thus the GC-MS and NMR data indicate that hydrogenation of the mixture of nepetalactone stereoisomers yielded the corresponding dihydronepetalactone enantiomers, as expected. The pair of enantiomers derived from *cis,trans*-nepetalactone (84.5 Mol% of the starting material) were the predominant dihydronepetalactones, at 78.6% of the mixture following hydrogenation.

## Claims

1. A non-therapeutic method of treating skin comprising applying thereto a dihydronepetalactone, or a mixture of dihydronepetalactone stereoisomers, having the general formula

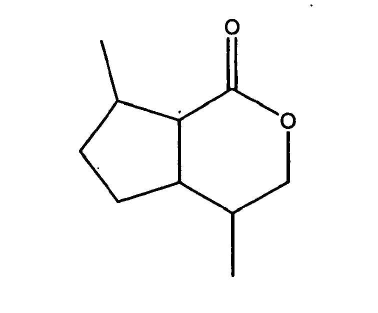

2. A method according to Claim 1 which comprises applying to skin a composition which comprises dihydronepetalactone and one or more of an adjuvant, carrier or fragrance compound that is not a dihydronepetalactone.

3. A method according to Claim 2 wherein the composition comprises dihydronepetalactone in an amount of from 0.01 % to 50% by weight of the total weight of the composition.

4. A method according to Claim 2 wherein the adjuvant, carrier or non-dihydronepetalactone fragrance compound is selected from the group consisting of alcohols, aldehydes, ketones, nitriles, esters, lactones, natural essential oils, synthetic essential oils, and mercaptans.

5. A method according to Claim 2 wherein the carrier comprises an absorbent solid or an encapsulation component.

6. A method according to any preceding Claim, wherein a dihydronepetalactone stereoisomeris one or more of:
(1S,9S,5R,6R) - 5-9-dimethyl-3-oxabicyclo [4.3.0] nonan-2-one;
(9S,5S,1R,6R) - 5-9-dimethyl-3-oxabicyclo [4.3.0] nonan-2-one; and
(95,1R,5R,6R)-5-9-dimethyl-3-oxabicyclo [4.3.0] nonan-2-one.

7. A method according to any preceding Claim, which comprises applying a lotion, powder, gel, foam, spray, cream or ointment to human skin.

8. A method according to any of Claims 2 to 6, wherein the composition is a fragrance or perfume composition.

9. A method according to any of claims 2 to 6, wherein the composition is a hair-care product.

## Patentansprüche

1. Nichttherapeutisches Verfahren zur Hautbehandlung, mit Aufbringen eines Dihydronepetalactons oder eines Gemischs von Dihydronepetalacton-Stereoisomeren mit der allgemeinen Formel 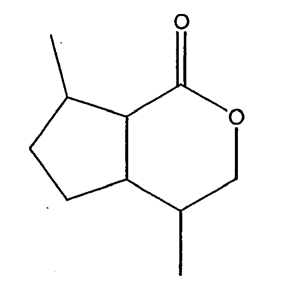 auf die Haut.

2. Verfahren nach Anspruch 1 mit Aufbringen einer Zusammensetzung auf die Haut, die Dihydronepetalacton und eine oder mehrere Komponenten aufweist, die unter einem Adjuvans, einem Träger oder einer Aromaverbindung, die kein Dihydronepetalacton ist, ausgewählt sind.

3. Verfahren nach Anspruch 2, wobei die Zusammensetzung Dihydronepetalacton in einem Anteil von 0,01 Gew.-% bis 50 Gew.-% des Gesamtgewichts der Zusammensetzung aufweist.

4. Verfahren nach Anspruch 2, wobei das Adjuvans, der Träger oder die Aromaverbindung, die kein Dihydronepetalacton ist, aus der Gruppe ausgewählt sind, die aus Alkoholen, Aldehyden, Ketonen, Nitrilen, Estern, Lactonen, natürlichen etherischen Ölen, synthetischen etherischen Ölen und Mercaptanen besteht.

5. Verfahren nach Anspruch 2, wobei der Träger einen absorbierenden Feststoff oder eine Verkapselungskomponente aufweist.

6. Verfahren nach einem der vorstehenden Ansprüche, wobei ein Dihydronepetalacton-Stereoisomer eine oder mehrere der folgenden Verbindungen ist:
(1S,9S,5R,6R)-5-9-Dimethyl-3-oxabicyclo[4.3.0]nonan-2-on;
(9S,5S,1R,6R)-5-9-Dimethyl-3-oxabicyclo[4.3.0]nonan-2-on und
(95,1R,5R,6R)-5-9-Dimethyl-3-oxabicyclo[4.3.0]nonan-2-on.

7. Verfahren nach einem der vorstehenden Ansprüche, welches das Aufbringen einer Lotion, eines Puders, Gels, Schaums, Sprays, einer Creme oder Salbe auf die menschliche Haut aufweist.

8. Verfahren nach einem der Ansprüche 2 bis 6, wobei die Zusammensetzung eine Aroma- oder Parfüm-Zusammensetzung ist.

9. Verfahren nach einem der Ansprüche 2 bis 6, wobei die Zusammensetzung ein Haarpflegemittel ist.

## Revendications

1. Méthode non thérapeutique pour traiter la peau comprenant l'application sur celle-ci d'une dihydronépétalactone, ou d'un mélange de stéréoisomères de dihydronépétalactone, ayant pour formule générale

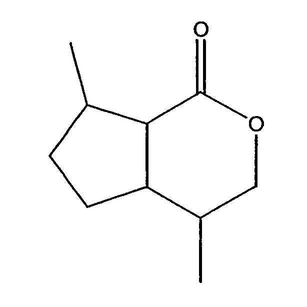

2. Méthode selon la revendication 1 qui comprend l'application sur la peau d'une composition qui comprend une dihydronépétalactone et un ou plusieurs éléments parmi un adjuvant, un véhicule ou un composé de fragrance qui n'est pas une dihydronépétalactone.

3. Méthode selon la revendication 2 dans laquelle la composition comprend la dihydronépétalactone en une quantité de 0,01 % à 50% en poids du poids total de la composition.

4. Méthode selon la revendication 2 dans laquelle l'adjuvant, le véhicule ou le composé de fragrance qui n'est pas une dihydronépétalactone est choisi dans le groupe constitué des alcools, des aldéhydes, des cétones, des nitriles, des esters, des lactones, des huiles essentielles naturelles, des huiles essentielles synthétiques, et des mercaptans.

5. Méthode selon la revendication 2 dans laquelle le véhicule comprend un solide absorbant ou un composant d'encapsulation.

6. Méthode selon l'une quelconque des revendications précédentes, dans laquelle un stéréoisomère de dihydronépétalactone est un ou plusieurs des composés suivants:
la (1S,9S,5R,6R)-5,9-diméthyl-3-oxabicyclo[4.3.0]nonan-2-one;
la (9S,5S,1R,6R)-5,9-diméthyl-3-oxabicyclo[4.3.0]nonan-2-one; et
la (9S,1R,5R,6R)-5,9-diméthyl-3-oxabicyclo[4.3.0]nonan-2-one.

7. Méthode selon l'une quelconque des revendications précédentes, qui comprend l'application d'une lotion, d'une poudre, d'un gel, d'une mousse, d'une pulvérisation, d'une crème ou d'une pommade sur de la peau humaine.

8. Méthode selon l'une quelconque des revendications 2 à 6, dans laquelle la composition est une composition de fragrance ou de parfum.

9. Méthode selon l'une quelconque des revendications 2 à 6, dans laquelle la composition est un produit de soin capillaire.
